# EUROPEAN PATENT APPLICATION

(11) **EP 4 306 514 A1**
(43) Date of publication of application: **17.01.2024**
(21) Application number: 22788467.3
(22) Date of filing: 14.04.2022
(51) Int. Cl.: C07D 231/56

(54) **METHOD FOR PREPARING INTERMEDIATE FOR SYNTHESIS OF SPHINGOSINE-1-PHOSPHATE RECEPTOR AGONIST**

(30) Priority: 14.04.2021 KR 20210048767
(71) Applicant: Lg Chem, Ltd., Seoul 07336 (KR)
(72) Inventor: KIM, Sung Wook, Daejeon 34122 (KR); KIM, Ki Dae, Daejeon 34122 (KR); LEE, Soo Min, Daejeon 34122 (KR); CHOI, In Hwan, Daejeon 34122 (KR)
(74) Representative: Hoffmann Eitle
(86) International application number: PCT/KR2022/005404
(87) International publication number: WO 2022/220612

(57) **Abstract**

The present invention relates to a novel method for preparing a compound represented by Formula 6 set forth in the present specification, wherein the compound can be effectively used for the synthesis of a sphingosine-1-phosphate receptor agonist.

## Description

### TECHNICAL FIELD

### Cross-reference to Related Applications

The present application claims the benefit of priority based on Korean Patent Application No. 10-2021-0048767, filed on April 14, 2021, the entire disclosure of which is incorporated as part of the specification.

### Technical Field

The present invention relates to a preparation method for major intermediates for synthesizing a sphingosine-1-phosphate receptor agonist.

### BACKGROUND ART

Sphingosine-1-phosphate (S1P) is produced via an intracellular ceramide pathway, in which ceramide is the starting material. Ceramide is produced via two pathways, the first of which is de novo biosynthetic pathway. Ceramide is also produced by the degradation of sphingomyelin, a cell membrane constituent, in a cell. The S1P level in each tissue is controlled by two biosynthetic sphingosine kinases (SphKs) and two biodegradable S1P phosphatases (S1P lyase and lysophospholipid phosphatases). S1P, produced via phosphorylation of sphingosine by sphingosine kinase, is known to mediate various cellular responses, such as cell proliferation, cytoskeletal organization and migration, adherence- and tight junction assembly, and morphogenesis. S1P exists as a combined form with other plasma proteins, including albumin, at a high level (100-1000 nM) in plasma, while it is at a low level in tissues.

S1P binds with the S1P receptor, a G-protein coupled receptor, to show various biological functions. As S1P receptor sub-types, S1P1-S1P5 are known up to now and are named endothelial differentiation gene (EDG) receptors 1, 5, 3, 6, and 8, respectively. The S1P receptors are known to be involved in various biological functions such as leukocyte recirculation, neural cell proliferation, morphological changes, migration, endothelial function, vasoregulation, and cardiovascular development.

In recent years, many studies have found that the S1P signaling process via these receptors plays an important role in a series of responses related to multiple sclerosis, including inflammation response and the repair process, and a non-selective S1P1 agonist has been recently and actually approved as a therapeutic agent for multiple sclerosis. The S1P receptors are extensively expressed in many cells related to the induction of multiple sclerosis. Especially, the S1P1 receptor plays a very important role in the immune system. The S1P1 receptor is mainly expressed on the surface of lymphocytes such as T cells and B cells and responds to S1P, resulting in involvement in recirculation of lymphocytes. In normal conditions, the S1P concentration is higher in body fluid than in lymphoid tissue, and therefore lymphocytes leave lymphoid tissue by the difference of S1P concentration to circulate along efferent lymph. However, when the S1P1 receptor in lymphocytes is down-regulated by an S1P1 agonist, the egress of lymphocytes from lymphoid tissue does not occur, resulting in reduced infiltration of autoaggressive lymphocytes, which cause inflammation and tissue damage in the central nervous system (CNS). As a result, a therapeutic effect on multiple sclerosis is obtained. Fingolimod, a non-selective S1P1 agonist, has been approved as an oral medication for treating multiple sclerosis. When it binds to the S1P1 receptor and is activated, the receptor becomes degraded or internalized from the surface of lymphocytes. Thus, fingolimod acts as a functional S1P1 antagonist paradoxically.

Concerning such an S1P receptor agonist, Korean Unexamined Publication No. 10-2014-0104376 discloses a novel compound of Formula 1, which is effective as an S1P receptor agonist: 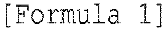 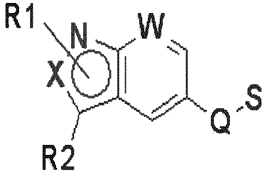 wherein
X is C or N,
R1 is H or a substitutable alkyl,
R2 is H, a substitutable alkyl, a halogen, CN, CF₃, or COCF₃,
W is C, N, C-alkoxy, C-halogen, or C-CN,
Q is CH₂O or 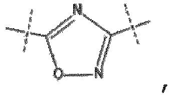
S is selected from among the following moieties:

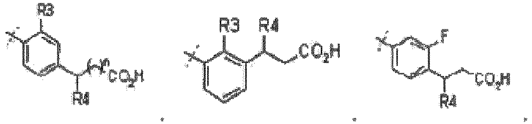

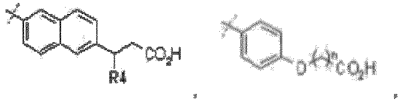

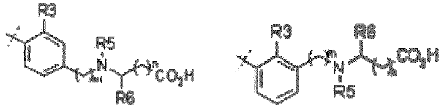

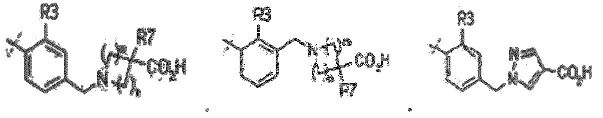

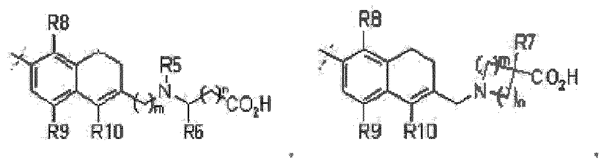

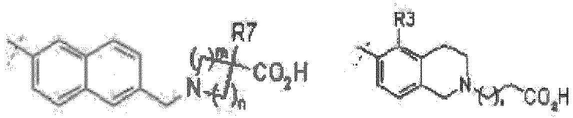

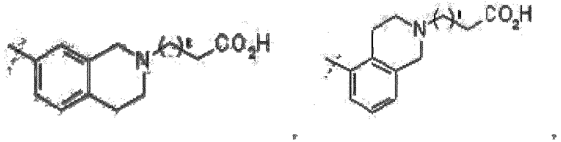

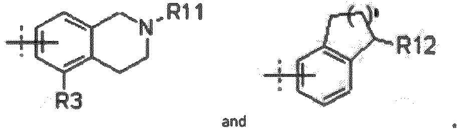

In the above structural formulae,
m and n are 0, 1, 2, or 3,
R3 to R10 are each H, an alkyl, a halogen, a halogenoalkyl, or an alkoxy alkyl,
R11 is H, 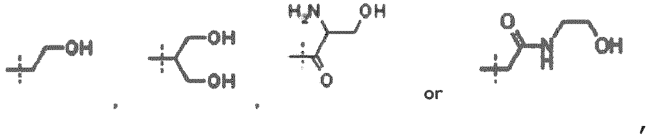 and
R12 is OH, NH₂,

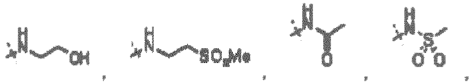

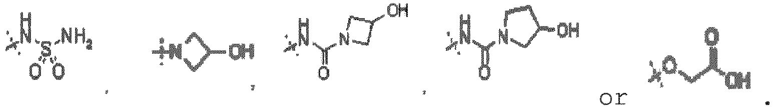

In a specific example of the above document, the preparation of 1-[1-chloro-6-(3-chloro-1-isopropyl-1H-indazol-5-ylmethoxy)-3,4-dihydro-naphthalen-2-ylmethyl]piperidine-4-carboxylic acid according to scheme 1 below is disclosed (in scheme 1, "SG35" refers to "1-chloro-6-hydroxy-3,4-dihydro-naphthalene-2-carbaldehyde") .

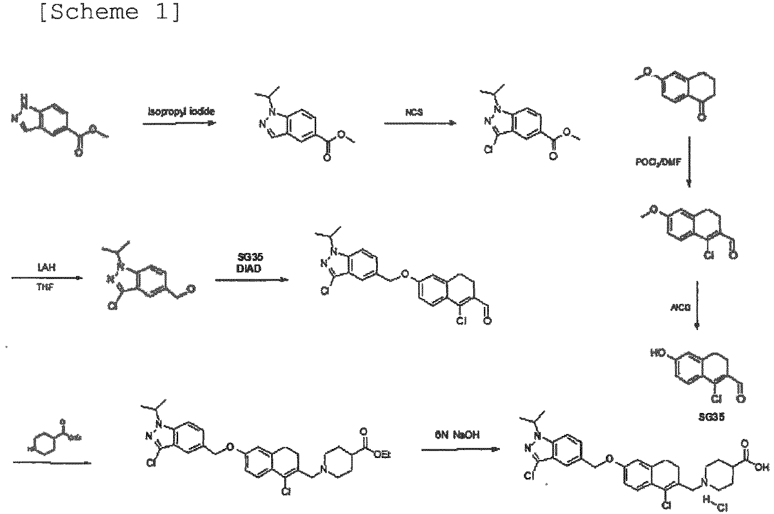

In scheme 1 above, a step for preparing 1-chloro-6-(3-chloro-1-isopropyl-1H-indazol-5-ylmethoxy)-3,4-dihydro-naphthalen-2-carbaldehyde is described in more detail as follows.

### (1-1) Synthesis of (3-chloro-1-isopropyl-1H-indazol-5-yl)-methanol

1H-Indazole-5-carboxylic acid methyl ester was dissolved in dimethylformamide, and isopropyl iodide and sodium hydride were slowly added dropwise at 0°C, followed by stirring at 50°C for 8 hours. 1 N hydrochloric acid solution was added, and extraction with ethyl acetate was carried out. The extract was washed with brine, dried with anhydrous magnesium sulfate and filtered. The filtrate was distilled under reduced pressure. Separation by column chromatography gave 1-isopropyl-1H-indazole-5-carboxylic acid methyl ester.

1-Isopropyl-1H-indazole-5-carboxylic acid methyl ester obtained above was dissolved in dimethylformamide, and N-chlorosuccinimide (NCS) was added dropwise, followed by stirring at room temperature for 18 hours. Water was added, and extraction with ethyl acetate was carried out. The extract was washed with brine, dried with anhydrous magnesium sulfate and filtered. The filtrate was distilled under reduced pressure. The residue was separated by column chromatography to obtain 3-chloro-1-isopropyl-1H-indazole-5-carboxylic acid methyl ester.

After the above-obtained 3-chloro-1-isopropyl-1H-indazole-5-carboxylic acid methyl ester was dissolved in tetrahydrofuran, lithium aluminum borohydride was added dropwise. After stirring at room temperature for 1 hour, water, 6N aqueous sodium hydroxide solution, and water were sequentially added. Celite was added dropwise, and the filtrate was distilled under reduced pressure. The residue was separated by column chromatography to obtain (3-chloro-1-isopropyl-1H-indazol-5-yl)-methanol.

### (1-2) Synthesis of 1-chloro-6-hydroxy-3,4-dihydronaphthalene-2-carbaldehyde

First, N,N-dimethylformamide (DMF) and phosphoryl chloride (phosphorous oxychloride, POCl₃) were added dropwise to a solution of 6-methoxy-3,4-dihydronaphthalen-1(2H)-one dissolved in toluene at 0°C, followed by stirring for 70°C for 6 hours. The reaction mixture was poured into ice, and extraction with ethyl acetate was carried out. The organic layer was washed with brine, dried and concentrated, and then the obtained residue was purified by silica gel column chromatography (hexane:ethyl acetate=20:1 to 10:1) to obtain 1-chloro-6-methoxy-3,4-dihydro-2-naphthalene carbaldehyde.

Next, aluminum chloride (AlCl₃) was added to a solution of 1-chloro-6-methoxy-3,4-dihydro-2-naphthalene carbaldehyde dissolved in dichloromethane at 0°C, followed by stirring at 50°C for 6 hours. The reaction mixture was poured into ice, and extraction with ethyl acetate was carried out. The organic layer was dried and concentrated, and then the obtained residue was purified by silica gel column chromatography (hexane:tetrahydrofuran = 5:1 to 3:1) to obtain 1-chloro-6-hydroxy-3,4-dihydro-2-naphthalene carbaldehyde.

### (1-3) Synthesis of 1-chloro-6-(3-chloro-1-isopropyl-1H-indazol-5-ylmethoxy)-3,4-dihydro-naphthalene-2-carbaldehyde

(3-Chloro-1-isopropyl-1H-indazol-5-yl)-methanol and 1-chloro-6-hydroxy-3,4-dihydro-2-naphthalene carbaldehyde, which were obtained above, were dissolved in toluene, and then tributyl phosphine (PBu₃) and 1,1'-(azodicarbonyl)dipiperidine (ADD) were added dropwise. After stirring the mixture at room temperature for 18 hours, an excess amount of hexane was added thereto. After filtration and distillation under reduced pressure, the residue was purified by column chromatography to obtain 1-chloro-6-(3-chloro-1-isopropyl-1H-indazol-5-ylmethoxy)-3,4-dihydro-naphthalene-2-carbaldehyde.

However, the above reactions may have the following problems in producing clinical APIs:

First, in the process of synthesizing 3-chloro-1-isopropyl-1H-indazole-5-carboxylic acid methyl ester, there may be a problem due to the production ratio of the N2 isomer. Lithium aluminum hydride (LAH) used for the synthesis of (3-chloro-1-isopropyl-1H-indazol-5-yl)-methanol has the following disadvantages: it has very limited stability for use in large scale synthesis and is easily decomposed by water.

In addition, during the Vilsmeier-Haack reaction to obtain 1-chloro-6-methoxy-3,4-dihydro-2-naphthalene carbaldehyde, the exothermic problem may arise as the reaction occurs at a high temperature of 70°C. In addition, in the reaction to obtain 1-chloro-6-hydroxy-3,4-dihydro-2-naphthalene carbaldehyde, there may be a problem of reactor contamination due to the use of AlCl₃ or a stability problem due to the use of hazardous reagents. When AlCl₃ is used, there is a stability problem due to the occurrence of batch fail caused by reaction stop or side reaction progress, and the total yield is 70%, so there is a need to improve the yield.

In addition, 1,1'- (azodicarbonyl)dipiperidine (ADD), which is used for the coupling of (3-chloro-1-isopropyl-1H-indazol-5-yl)-methanol and 1-chloro-6-hydroxy-3,4-dihydronaphthalene-2-carbaldehyde, is not preferable in terms of a low yield problem and cost.

Accordingly, to mass-produce an intermediate compound, such as 1-chloro-6-(3-chloro-1-isopropyl-1H-indazol-5-ylmethoxy)-3,4-dihydro-naphthalene-2-carbaldehyde, with high yield through a simpler process, the inventors of the present invention has invented a new synthesis method as shown in scheme 2 below.

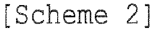

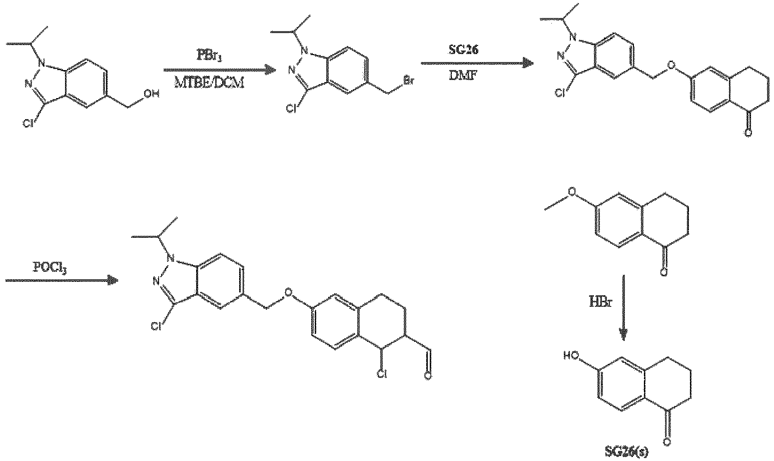

In scheme 2 above, a step for preparing 1-chloro-6-(3-chloro-1-isopropyl-1H-indazol-6-ylmethoxy)-3,4-dihydro-naphthalen-2-carbaldehyde is described in more detail as follows (in scheme 2, "SG26" refers to "6-hydroxy-3,4-dihydro-2H-naphthalen-1-one").

### (2-1) Synthesis of 5-bromomethyl-3-chloro-1-isopropyl-1H-indazole

Dichloromethane (DCM), methyl tert-butyl ether (MTBE), and (3-chloro-1-isopropyl-1H-indazol-5-yl)-methanol were placed into the reactor, and cooling to the internal temperature of 0°C was performed. PBr₃ was slowly added dropwise to the reaction for 70 minutes, and then the reaction was allowed to proceed for 80 minutes. Ion-pair chromatography (IPC) was performed using HPLC and the reaction was completed (3% > (3-chloro-1-isopropyl-1H-indazol-5-yl)-methanol). The reaction was terminated by slowly adding sodium hydroxide for 120 minutes. DCM was added to the reaction mixture, which was stirred for 30 minutes. The layers were separated, the aqueous layer was removed, and the organic layer was washed with water. Then, the organic layer was distilled under reduced pressure to obtain 5-bromomethyl-3-chloro-1-isopropyl-1H-indazole.

### (2-2) Synthesis of 6-hydroxy-3,4-dihydro-2H-naphthalen-1-one

HBr and 6-methoxy-3,4-dihydro-2H-naphthalen-1-one, which were dissolved in water, were placed into the reactor, and the reaction was refluxed at an external temperature of 120°C for 52 hours. IPC was carried out using HPLC and the reaction was completed (3% > 6-methoxy-3,4-dihydro-2H-naphthalen-1-one). Cooling to the internal temperature of 10°C was performed, and the resulting solid was filtered. After washing with water and drying with nitrogen, 6-hydroxy-3,4-dihydro-2H-naphthalen-1-one (SG26) was obtained.

### (2-3) Synthesis of 6-(3-chloro-1-isopropyl-1H-indazol-5-ylmethoxy)-3,4-dihydro-2H-naphthalen-1-one

5-Bromomethyl-3-chloro-1-isopropyl-1H-indazole, 6-hydroxy-3,4-dihydro-2H-naphthalen-1-one, K₂CO₃, and DMF were placed into the reactor and reacted at the internal temperature of 25°C for 3 hours. IPC was performed using HPLC. 5% of 6-hydroxy-3,4-dihydro-2H-naphthalen-1-one remained. So, 5-bromomethyl-3-chloro-1-isopropyl-1H-indazole was additionally added to complete the reaction (1% > 6-hydroxy-3,4-dihydro-2H-naphthalen-1-one). Water was added to the reactor, cooling to the internal temperature of 0°C was performed, and then the resulting solid was filtered. The filtered solid was washed sequentially with water and MTBE, respectively, and then dried with nitrogen to obtain 6-(3-chloro-1-isopropyl-1H-indazol-5-ylmethoxy)-3,4-dihydro-2H-naphthalen-1-one.

### (2-4) Synthesis of 1-chloro-6-(3-chloro-1-isopropyl-1H-indazol-6-ylmethoxy)-3,4-dihydro-naphthalene-2-carbaldehyde

Phosphoryl chloride (POCl₃) was placed into the reactor, and cooling to the internal temperature of 0°C was performed. DMF was slowly added dropwise, stirring at the internal temperature of 50°C was performed for 2 hours, and 6-(3-chloro-1-isopropyl-1H-indazol-5-ylmethoxy)-3,4-dihydro-2H-naphthalene-1-one was added and reacted at the internal temperature of 50°C for 3 hours. Excessive HCl gas is generated during the reaction. So, a vent line was installed so that excessive HCl gas could be neutralized by a NaOH trap. IPC was carried out using HPLC and the reaction was completed. Cooling to the internal temperature of 0°C was performed, and then cold water, hexane (Hex), and MTBE were added to another reactor. The above reaction mixture was slowly added dropwise for 90 minutes to form crystals. The resulting solid was filtered, washed sequentially with water and a mixed solvent of MTBE/HEX, respectively, and dried to obtain 1-chloro-6-(3-chloro-1-isopropyl-1H-indazol-6-ylmethoxy)-3,4-dihydronaphthalene-2-carbaldehyde.

With the above scheme, the production rate of the N2 isomer can be improved, the exothermic problem caused by the Vilsmeier-Haack reaction can be resolved, and a coupling reaction can be performed without ADD. Therefore, it is expected that the key intermediate for sphingosine-1-phosphate receptor agonist synthesis can be mass-produced through a simpler process while ensuring the stability of the compound and the stability of the manufacturing conditions.

However, since it is still necessary to further reduce impurities during the API process of the material finally produced by the above synthesis method, research on each step of the above synthesis method is continuously required.

### DISCLOSURE OF THE INVENTION

### TECHNICAL PROBLEM

Thus, an aspect of the present invention provides a suitable method for producing a compound of Formula 4, which is a key intermediate in a novel synthesis method of an excellent sphingosine-1-phosphate receptor agonist, with high yield and high purity: 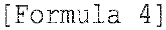 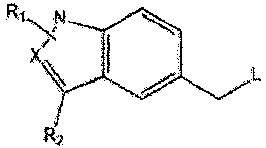 wherein
R1 is hydrogen or a substituted or unsubstituted alkyl,
R2 is hydrogen, a substituted or unsubstituted alkyl, a halogen, CN, CF₃, or COCF₃,
X is C or N, and
L is a leaving group.

### TECHNICAL SOLUTION

According to an aspect of the present invention, there is provided a method for preparing an intermediate compound of Formula 4 below, comprising: 1) a step for performing a reduction reaction of a compound of Formula 2 in the presence of an alcohol-based solvent and an alkoxide to obtain a compound of Formula 3; and 2) a step for substituting the alcohol group of the compound of Formula 3 with a leaving group: 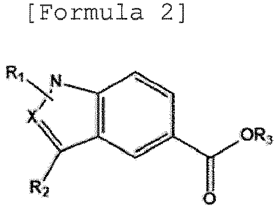 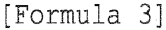 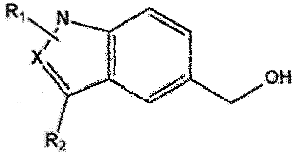 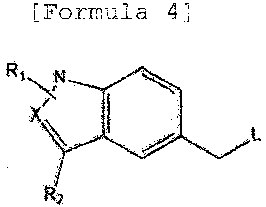 wherein
R1 is hydrogen or a substituted or unsubstituted alkyl,
R2 is hydrogen, a substituted or unsubstituted alkyl, a halogen, CN, CF₃, or COCF₃,
R3 is a substituted or unsubstituted alkyl,
X is C or N, and
L is a leaving group.

When the 'alkyl' is substituted, there may be one or more substituents, and the substituents may be each independently selected from the group consisting of a halogen, cyano, hydroxy, alkyloxy, oxo, an unsubstituted sulfonyl, and a sulfonyl substituted with an alkyl.

According to one embodiment of the present invention, R1 of the above formulae may be hydrogen or a C₁-C₆ substituted or unsubstituted alkyl, and R2 may be hydrogen, a C₁-C₆ substituted or unsubstituted alkyl, a halogen, CN, CF₃, or COCF₃. R3 may be a C₁-C₆ substituted or unsubstituted alkyl.

According to another embodiment of the present invention, R1 may be a C₁-C₄ substituted or unsubstituted alkyl, and R2 may be a halogen (F, Cl, Br, or I). R3 may be a C₁-C₄ substituted or unsubstituted alkyl, for example, methyl.

According to one embodiment of the present invention, the leaving group (L) is a reactive group that provides a substitution position to the compound of Formula 4 when the compound of Formula 4 is subjected to a substitution reaction with an alcohol-based compound, and may be, without limitation, selected from among chlorine (Cl), bromine (Br), iodine (I), methanesulfonate (Oms), p-toluenesulfonate (OTs), and trifluoromethanesulfonate (OTf).

According to another embodiment of the present invention, L may be Br.

In the present invention, in step 1), a reduction reaction of the compound of Formula 2 is performed in the presence of an alcohol-based solvent and an alkoxide to obtain the compound of Formula 3.

Specifically, step 1) aims to reduce the ester group of the compound of Formula 2 to an alcohol.

For the reduction reaction of the compound of Formula 2, a reducing agent commonly used for the reduction of an ester group to an alcohol may be used. The reducing agent may be, but is not limited to, at least one selected from among sodium borohydride (NaBH₄), lithium borohydride (LiBH₄), borane (BH₃), and diisobutylaluminum hydride (DIBAH).

The reduction reaction in step 1) may be performed in a polar solvent to provide high solubility to the compound of Formula 2 and the reducing agent. For example, the reduction reaction may be performed in water, a polar organic solvent, or a mixed solvent thereof.

In the present invention, the reaction solvent for the reduction reaction in step 1) includes an alcohol-based solvent. The alcohol-based solvent may be appropriately selected and used depending on the compound of Formula 2 and the reducing agent. For example, at least one selected from among methanol, ethanol, n-propane alcohol, isopropane alcohol, n-butanol, sec-butanol, iso-butanol, and tert-butanol may be used, but the present invention is not limited thereto.

In one embodiment of the present invention, the reaction solvent for the reduction reaction in step 1) may include methanol.

In another embodiment of the present invention, the reaction solvent for the reduction reaction in step 1) may include water and tetrahydrofuran (THF).

In the present invention, the reducing agent used in the reduction reaction may generate hydrogen in a polar solvent, such as an alcohol-based solvent, thereby causing a problem of reducing the stability of the reaction, or may induce over-reduction of the compound of Formula 2 to further cause the reduction of an olefin group, thereby causing a problem of forming impurities.

Accordingly, the present invention provides a preparation method for stabilizing the reduction reaction by using an alkoxide as an additive in the reaction in step 1) and reducing the formation of impurities by suppressing over-reduction.

In one embodiment of the present invention, the alkoxide may be selected and used independently of the alcohol-based solvent, or an alkoxide corresponding to the alcohol-based solvent may be selected and used.

In another embodiment of the present invention, the alkoxide may include one having the same alkyl group as the alkyl group of the alcohol-based solvent.

In another embodiment of the present invention, the alcohol-based solvent may include methanol, and the alkoxide may include methoxide.

In one embodiment of the present invention, the reducing agent used in the reduction reaction may include a metal cation, wherein the metal ion in the alkoxide may be different from/identical to that of the reducing agent.

In another embodiment of the present invention, the alkoxide may include the same metal ion as the metal ion within the reducing agent used in the reduction reaction.

In another embodiment of the present invention, in step 1), the compound of Formula 3 may be obtained by performing a reduction reaction of the compound of Formula 2 in the presence of methanol solvent and sodium methoxide.

According to one embodiment of the present invention, in the reduction reaction of the compound of Formula 2, the reducing agent and the solvent may be added together at the beginning of the reaction, and then the reducing agent may be additionally added as the reaction proceeds. When additionally adding the reducing agent, the solvent added at the beginning of the reaction may be added together, or only the reducing agent may be added without adding the solvent.

According to another embodiment of the present invention, by using the alkoxide as an additive in step 1), the reduction reaction may be terminated using only the initially added reducing agent without additionally adding the reducing agent during the reduction reaction so that the process stability may be improved. But the present invention is not limited thereto.

According to another embodiment of the present invention, by using the alkoxide as an additive in step 1), the reduction reaction may be performed at a temperature of 60°C or less, for example, 40°C to 55°C, 45°C to 50°C, so that the process temperature may be lowered. But the present invention is not limited thereto.

In one embodiment of the present invention, the content of the alkoxide in step 1) may be, for example, 1 to 5 mol%, specifically 2 to 3 mol%, and more specifically 2.5 mol%. Using the alkoxide in the above content can improve the purity of a product and the process efficiency. But the effect of the present invention is not limited thereto.

In another embodiment of the present invention, the amount of the alcohol-based solvent used in step 1) may be, for example, 0.1-fold to 5-fold, for example, 0.4-fold to 3.6-fold, 1-fold to 3-fold, 1.5-fold to 2-fold, or 1.6-fold, compared to the compound of Formula 2. Using the alcohol-based solvent in the above amount can improve the purity of a product and the process efficiency. But the effect of the present invention is not limited thereto.

In another embodiment of the present invention, the amount of the reducing agent used in step 1) may be, for example, 1 to 5 equivalents, 1.4 to 4.6 equivalents, 2 to 4 equivalents, or 3 equivalents. Using the reducing agent in the above amount can improve the purity of a product and the process efficiency. In particular, in performing the reduction process, using the reducing agent in the above amount can enable the reaction to be completed without further addition of the reducing agent during the process, after the addition of the reducing agent at the beginning of the reaction. Thus, the process stability can be greatly improved. But the effect of the present invention is not limited thereto.

In another embodiment of the present invention, in step 1), the reduction reaction may be performed using 3 equivalents of NaBH₄ in the presence of the 1.6-fold amount of methanol solvent and 2.5 mol% of sodium methoxide.

In the present invention, step 1) may further include purifying to improve the purity of a product.

The purifying may be performed under an acidic condition to remove impurities formed by over-reduction during the reduction reaction of step 1).

The acidic condition may be formed, for example, by adding an acid compound to a reaction product, wherein the acid compound may be, but is not limited to, at least one selected from hydrogen chloride (HCl) and hydrogen bromide (HBr).

In one embodiment of the present invention, the purifying in step 1) may be performed by adding an acid compound to a reaction product and then extracting an organic layer.

In another embodiment of the present invention, the purifying in step 1) may be performed by washing a reaction product, adding HCl, stirring, and then extracting an organic layer.

In one embodiment of the present invention, hydrogen chloride to be added may be used in 3 N to 8 N, for example, 5 N to 7 N, or 6 N.

In one embodiment of the present invention, the purifying may be performed at a temperature of 0°C to 20°C.

In another embodiment of the present invention, the purifying may be performed while maintaining 0°C.

In one embodiment of the present invention, the purifying may be performed by additionally adding an organic solvent to a reaction product and then extracting an organic layer.

The solvent for extracting an organic layer may be appropriately selected and used as needed. For example, toluene, ethyl acetate, methyl tertiary butyl ether, tetrahydrofuran, methanol, dichloromethane, or a mixture thereof may be used. But the present invention is not limited thereto.

In one embodiment of the present invention, by additionally adding toluene to a reaction product, the purifying may be performed in the presence of an organic solvent containing toluene. When adding toluene to a reaction product, adding an acid compound, and then extracting an organic layer, the purity of a reaction product can be effectively increased, but the effect of the present invention is not limited thereto.

In another embodiment of the present invention, in the purifying, the procedure of adding toluene to a reaction product to dissolve a residue, washing with water, adding hydrogen chloride to an organic layer, stirring, and then removing an aqueous layer may be performed at least once, for example, twice.

In the present invention, in step 2), the alcohol group of the compound of Formula 3 is substituted with a leaving group.

In the present invention, a compound of Formula 4, a key intermediate in the sphingosine-1-phosphate receptor agonist synthesis, is obtained by substituting the terminal alcohol group of the compound of Formula 3 with a leaving group. The inclusion of a leaving group as in the compound of Formula 4 can improve the yield of the coupling reaction in the subsequent sphingosine-1-phosphate receptor agonist synthesis.

In one embodiment of the present invention, the 'step for substituting the alcohol group with a leaving group' (hereinafter referred to as 'leaving group substitution step') may be performed in the presence of a polar organic solvent.

In another embodiment of the present invention, the leaving group substitution step may be performed in the presence of DCM and MTBE.

In another embodiment of the present invention, the leaving group substitution step may be performed in an ether-based single solvent. Performing the leaving group substitution step in an ether-based single solvent can significantly lower the production rate of the N2 isomer. But the effect of the present invention is not limited thereto.

The 'single solvent' indicates that only one kind of solvent is included in a reactor for the leaving group substitution reaction. The inclusion of a heterogeneous solvent in a trace amount corresponding to a level at which the yield of a reaction product is not substantially affected in the reactor for the leaving group substitution reaction is also not excluded from the single solvent. For example, the inclusion of a heterogeneous solvent in a content of 5 vol% or less, 4 vol% or less, 3 vol% or less, 2 vol% or less, 1 vol% or less, 0.5 vol% or less, or 0 vol% (i.e., no inclusion) based on the total volume of a solvent for the leaving group substitution reaction can be said as the use of the single solvent.

Examples of the ether-based solvent include, but are not limited to, dialkyl ether-based solvents, such as diethyl ether, dipropyl ether, dibutyl ether, diisoamyl ether, ethyl methyl ether, methyl propyl ether, methyl butyl ether, and ethyl propyl ether; aryl allyl ether-based solvents, such as diphenyl ether and anisole; or cyclic ether-based solvents, such as tetrahydrofuran and tetrahydropyran; and so on.

In one embodiment of the present invention, the ether-based single solvent may be methyl tert-butyl ether (MTBE).

In another embodiment of the present invention, the compound of Formula 4 may be obtained by mixing the compound of Formula 3 and MTBE, cooling to 0°C, and reacting the compound of Formula 3 with PBr₃.

In another embodiment of the present invention, the compound of Formula 4 may be obtained by mixing the compound of Formula 3 and MTBE, cooling to 0°C, reacting the compound of Formula 3 with PBr₃, and on completion of the reaction, washing with water and performing the filtration.

In another aspect, according to the present invention, the compound of Formula 2 may be prepared by introducing R1 and R2 substituents to a compound of Formula 5: 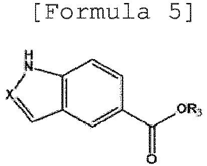 wherein R1, R2, R3, and X are as defined in Formula 2 above.

The substitution of R1 and R2 may be in the following order: R1 substitution and then R2 substitution, R2 substitution and then R1 substitution, or simultaneous substitution of R1 and R2.

In one embodiment of the present invention, in the compound of Formula 5, R2 may be substituted before R1. When bulky R1 is first substituted in the compound of Formula 5, for example, when bulky R1 is first substituted at position 3 of indazole in which X is N, the production of the N2 isomer can be inhibited, and the yield can be improved.

The reactions for introducing R1 and R2 substituents may be performed in the same solvent or different solvent compositions.

In one embodiment of the present invention, the reactions for introducing R1 and R2 substituents may be performed in the same solvent.

In one embodiment of the present invention, the reaction solvent of the reactions for introducing R1 and R2 substituents may include, for example, an amide-based organic solvent. The amide-based organic solvent may include, for example, at least one selected from dimethylformamide (DMF) and dimethylacetamide (DMA).

In another embodiment of the present invention, the reaction solvent of the reactions for introducing R1 and R2 substituents may include dimethylacetamide.

In another embodiment of the present invention, the reaction solvent of the reactions for introducing R1 and R2 substituents may be dimethylacetamide alone.

In the reaction for introducing R1 and R2 substituents, when the reaction solvent is unstable and thus decomposed, the decomposition product may react with the reactant of the synthesis reaction to induce the release of heat, thereby generating an excess of impurities and reducing the purity of the reaction product. In this regard, since the maximum temperature of the synthesis reaction and the degree of adiabatic temperature arise for the synthesis reaction in which dimethylformamide is used as the reaction solvent are higher than those for the synthesis reaction in which dimethylacetamide is used as the reaction solvent, it may be preferable to use dimethylacetamide as the reaction solvent for the synthesis reaction.

In one embodiment of the present invention, when the synthesis reaction is performed in dimethylformamide solvent, the release of heat may be induced due to the reaction between dimethylamine formed by decomposition of dimethylformamide and N-chlorosuccinimide (NCS) used to introduce Cl at the R2 position, and an impurity in which Cl is introduced at a position other than R2 in the compound of Formula 5 may be formed by an increase in the internal temperature. Accordingly, there may be a problem of reducing the purity of the compound of Formula 2 and a subsequent process.

In one embodiment of the present invention, the compound of Formula 2 may be prepared by introducing R1 and R2 substituents to the compound of Formula 5 in the step for introducing R1 and R2 and performing the crystallization using a crystallization solvent including an alcohol solvent.

The 'alcohol solvent' for the crystallization may be, without limitation, one or more solvents selected from among methanol, ethanol, isopropyl alcohol, and butanol, for example.

In one embodiment of the present invention, the solvent for the crystallization may be a mixed solvent of an alcohol solvent and water. Using a mixed solvent of an alcohol solvent and water as the crystallization solvent can reduce the yield of the N2 isomer.

In another embodiment of the present invention, for the mixed solvent for crystallization, the volume ratio of the alcohol solvent and water may be 5:1 to 1:5, 4:1 to 1:4, 3:1 to 1:3, 2:1 to 1:2, 2:1 to 1:1, or 1.5:1 to 1:1, in terms of the yield of Formula 5.

In one embodiment of the present invention, the solvent for the crystallization may be a mixed solvent of ethanol and water. Specifically, ethanol and water may be used in a volume ratio of 2:1 to 1:2, 2:1 to 1:1, 1.5:1 to 1:1, or 1:1 of EtOH:H₂O.

In another embodiment of the present invention, when the crystallization solvent is a mixed solvent of an alcohol solvent and water, the alcohol solvent and water may be added sequentially or simultaneously during the crystallization.

In another embodiment of the present invention, the compound of Formula 5 may be obtained by adding an alcohol solvent, such as EtOH, to the reaction product in which the substituents have been introduced, cooling to 0°C to 20°C, or adding water at room temperature to perform the crystallization.

In one embodiment of the present invention, before the crystallization, the reaction product in which the substituents have been introduced may be purified and then crystallized. Purifying the reaction product can remove unreacted residual compounds used in the reaction and thus improve the crystallization yield.

The purification may be performed using, for example, a polar solvent, and the polar solvent may be, for example, a polar organic solvent, water, or a mixed solvent thereof.

The polar organic solvent may be, without limitation, one or more solvents selected from among ethyl acetate (EA), isopropyl acetate (IPOAc), dichloromethane, and hexane.

In one embodiment, according to the present invention, purifying the reaction product in which the substituents have been introduced using a mixed solvent of the polar organic solvent and water, and then performing the crystallization can prevent K₂CO₃ used in the reaction for introducing the substituents from precipitating during the crystallization of the reaction product or reduce the precipitation amount, thereby improving the purity of the crystals.

The compound prepared according to the present invention can be used as a key intermediate for sphingosine-1-phosphate receptor agonist synthesis. The compound prepared according to the present invention can be used as a key intermediate for a known synthesis method for a sphingosine-1-phosphate receptor agonist and can also be used as a key intermediate for a new synthesis method to be developed after the present application. However, the use of the present invention is not limited to a particular method for synthesizing a sphingosine-1-phosphate receptor agonist.

In addition, the compound prepared according to the present invention can be used for other purposes other than the sphingosine-1-phosphate receptor agonist synthesis, and the use of the present invention is not limited to the sphingosine-1-phosphate receptor agonist synthesis.

### ADVANTAGEOUS EFFECTS

Using the preparation method of the present invention can lead to mass production of the compound of Formula 4 with high purity and high yield.

### MODE FOR CARRYING OUT THE INVENTION

Hereinafter, to help the understanding of the present invention, the present invention will be described in more detail by examples. However, examples according to the present invention may be modified into various other types, and the scope of the present invention should not be construed as being limited to the following examples. The examples of the present invention are provided for completely explaining the present invention to a person having an average knowledge in the art to which the present invention belongs.

As an intermediate compound for the sphingosine-1-phosphate receptor agonist synthesis, 5-bromomethyl-3-chloro-1-isopropyl-1H-indazole was synthesized according to Scheme 3 below.

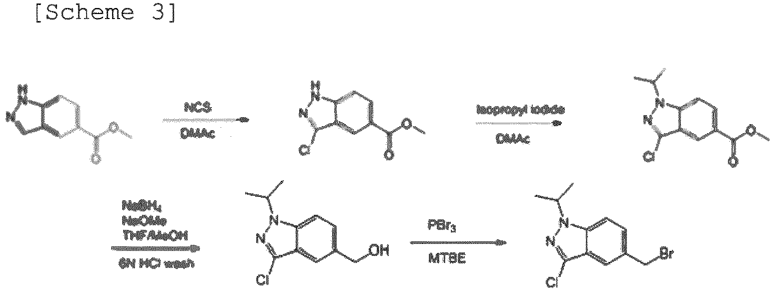

### Example 1-1. Synthesis of 3-chloro-1-isopropyl-1H-indazole-5-carboxylic acid methyl ester

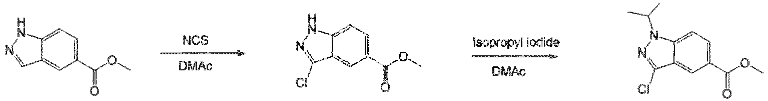

1H-Indazole-5-carboxylic acid methyl ester (1.0 kg, 6.58 mol) and N-chlorosuccinimide (NCS, 0.95 kg, 7.10 mol (1.25 equiv)) were dissolved in 5 L (5-fold) of dimethylacetamide (DMA), and then the reaction was allowed to proceed at 55°C for 1 hour.

The HPLC analysis showed that 1.19% of 1H-indazole-5-carboxylic acid methyl ester remained, and thus the reaction solution was cooled to 42°C. After adding 2-iodopropane (1.83 kg) and K₂CO₃ (2.35 kg) to the reaction, the temperature was raised to 55°C, and the alkylation reaction was performed for 3 hours.

The IPC analysis showed that methyl 3-chloro-1H-indazole-5-carboxylate remained, so 2-iodopropane (0.29 kg, total amount used 2.2 equiv., 12.49 mol) and K₂CO₃ (0.35 kg, total amount used 3.44 equiv., 19.53 mol) were additionally added, and the reaction was allowed to proceed for additional 2 hours. The IPC analysis showed that 1.45% of methyl 3-chloro-1H-indazole-5-carboxylate remained, and thus the reaction was cooled to room temperature.

7 L of IPOAc and 10 L of water were added to the reaction solution, the mixture was stirred for 10 minutes, the layers were separated to remove the aqueous layer, and additional washing was performed with 5 L of water to remove DMA. Distillation under reduced pressure was carried out to remove IPOAc, 4 L of EtOH was added to the residue to dissolve, and then 4 L of water was slowly added dropwise at room temperature to perform the crystallization. The title compound (1.32 kg, net yield 81%) was synthesized by aging the resulting solid for 2 hours, performing the filtration, washing twice with 7 L and 5 L of water, and drying with nitrogen.

¹H NMR (400 MHz, CDCl₃) : 1.58 (d, 6H), 3.96 (s, 3H), 4.81 (m, 1H), 7.42 (d, 1H), 8.06 (dd, 1H), 8.44 (s, 1H).

### Example 1-2. Synthesis of (3-chloro-1-isopropyl-1H-indazol-5-yl)-methanol

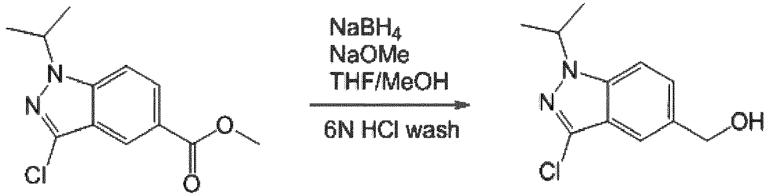

3-Chloro-1-isopropyl-1H-indazole-5-carboxylic acid methyl ester (Example 1-1, 0.97 kg, 3.84 mol) was placed into the reactor, dissolved in 5.8 L (6-fold) of THF, and then heated to 60°C. NaBH₄ (0.36 kg, 10.0 mol) and NaOMe (18 ml, 0.1 mol) were added to the reaction mixture, 1 L of MeOH was slowly added dropwise to the reaction solution, and then the reaction was allowed to proceed for 3 hours.

The IPC was carried out using HPLC, and the criterion (2% or less of 3-chloro-1-isopropyl-1H-indazole-5-carboxylic acid methyl ester) was satisfied to complete the reaction. The reaction solution was cooled to 10°C or less, quenching was performed by slowly adding 4.5 L of 3 N HCl dropwise, and distillation under reduced pressure was performed to remove THF and MeOH. 6.8 L of toluene was added to dissolve the residue, and washing was performed twice with 7.8 L and 6.8 L of water.

The title compound (0.73 kg, net yield 85%) was synthesized by repeating twice the procedure (of adding 6.7 L of 6 N HCl to the organic layer, stirring for 0.5 hours, and separating layers to remove the aqueous layer) to remove impurities (RRT0.88, RRT 0.91), and performing distillation under reduced pressure.

¹H NMR (400 MHz, CDCl₃): 1.5-1.7 (m, 6H), 1.82 (m, 1H), 3.72 (m, 1H), 4.70-5.10 (m, 2H), 7.30-7.50 (m, 2H), 7.62 (s, 1H) .

### Example 1-3: Synthesis of 5-bromomethyl-3-chloro-1-isopropyl-1H-indazole

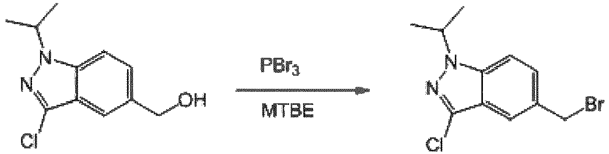

5.86 L of MTBE and (3-chloro-1-isopropyl-1H-indazol-5-yl)-methanol (Example 1-2, 0.73 kg, 3.26 mol) were placed into the reactor and cooling to the internal temperature of 0°C was performed. PBr₃ (0.53 kg, 1.96 mol) was slowly added to the reaction for 90 minutes, and the reaction was allowed to proceed for 180 minutes.

IPC was carried out using HPLC, and the reaction was completed ((3-chloro-1-isopropyl-1H-indazol-5-yl)-methanol: N/D). 1.5 N NaOH (5.9 L) was slowly added for 60 minutes, and stirring for 30 minutes was performed to complete the reaction. The title compound (0.84 kg, net yield 90.0%) was synthesized by adding brine (3.7 L) to the reaction mixture, stirring for 10 minutes, separating layers to remove the aqueous layer, additionally washing with brine (3.7 L), and distilling the organic layer under reduced pressure.

1H NMR (400 MHz, CDCl3): 1.53 (d, 6H), 4.7 (s, 2H), 4.88 (m, 1H), 7.51-7.6 (m, 2H), 7.68 (s, 1H).

### Experimental Example 1. Evaluation of purity improvement of the product depending on the use of alkoxide additive in the reduction reaction of (3-chloro-1-isopropyl-1H-indazol-5-yl)-methanol

According to the conventional process, in the reduction reaction of (3-chloro-1-isopropyl-1H-indazol-5-yl)-methanol, a problem of bubble rise to the top of the reactor due to hydrogen gas generated from using the reducing agent NaBH₄ was observed. In addition, there was a problem in that stability of the process was not secured because NaBH₄ had to be added about three to four times in the middle of the process until the end of the reduction reaction.

It was confirmed that these problems could be solved by using sodium methoxide (NaOMe) as a catalyst to control the decomposition of NaBH₄ and remove impurities (RRT 0.88IMP: compound in which the olefin group of indole is further reduced) generated during the reaction.

Accordingly, the degrees of the reaction completion and the impurities' generation depending on the amounts of the alkoxide additive used and the reaction temperatures were evaluated and are shown in Tables 1 and 2 below.

In Tables 1 and 2 below, "SG10" represents "3-chloro-1-isopropyl-1H-indazole-5-carboxylic acid methyl ester", and "SG15" represents "(3-chloro-1-isopropyl-1H-indazol-5-yl)-methanol".

**[Table 1]**

| Ent ry | NaOM e (mol %) | NaBH ₄ (equ iv) | MeOH (fol d) | Temp (°C) | Time (h) | SG15 HPLC (% PAR) | | | | | |
|---|---|---|---|---|---|---|---|---|---|---|---|
| | | | | | | RRT 0.88 | N2 SG15 | SG15 | SG10 | 14.7 Min | RRT 1.44 |
| 1 | 1 | 2 | 1.5 | 50 | 1 | 0.20 | 7.31 | 43.48 | 42.68 | 0.23 | 0.29 |
| | | | | | 16 | 0.18 | 7.26 | 43.43 | 42.86 | 0.20 | 0.28 |
| | | + 0.7 | + 0.3 | | 20 | 0.13 | 10.6 3 | 60.62 | 24.17 | 0.32 | 0.28 |
| 2 | 2.5 | 2 | 1.5 | 50 | 1 | 0.06 | 9.67 | 55.56 | 29.48 | 0.60 | 0.28 |
| | | | | | 16 | 0.05 | 9.62 | 55.62 | 29.51 | 0.55 | 0.28 |
| | | + 0.7 | + 0.3 | | 20 | 0.04 | 11.4 0 | 63.91 | 20.16 | 0.90 | 0.29 |
| 3 | 5 | 2 | 1.5 | 50 | 1 | 0.04 | 9.51 | 54.62 | 28.68 | 2.73 | 0.26 |
| | | | | | 16 | 0.05 | 9.49 | 54.65 | 28.80 | 2.57 | 0.27 |
| | | + 0.7 | + 0.3 | | 20 | 0.03 | 11.4 2 | 63.65 | 18.47 | 3.19 | 0.27 |

**[Table 2]**

| Entr y | NaBH₄ (equi v) | MeOH (fold ) | Temp (°C) | SG15 HPLC (% PAR) | | | | | |
|---|---|---|---|---|---|---|---|---|---|
| | | | | 0.88 | N2 SG15 | SG15 | N2 SG10 | SG10 | 14.7 min |
| 1 | 3 | 0.4 | 50 | 0.03 | 5.77 | 39.46 | 6.31 | 44.51 | 3.80 |
| 2 | 3 | 2 | 50 | 0.04 | 16.42 | 81.78 | 0.18 | 0.37 | 1.21 |
| 3 | 3 | 2 | 35 | 0.02 | 15.40 | 79.96 | 0.68 | 2.96 | 0.99 |
| 4 | 3 | 2 | 65 | 0.04 | 16.39 | 79.05 | 0.35 | 0.98 | 3.18 |
| 5 | 4.6 | 2 | 50 | 0.04 | 17.27 | 81.63 | 0.01 | 0.02 | 0.96 |
| 6 | 3 | 2 | 50 | 0.03 | 15.96 | 80.17 | 0.62 | 2.13 | 1.11 |
| 7 | 3 | 3.6 | 50 | 0.01 | 8.03 | 45.20 | 4.97 | 39.53 | 2.12 |
| 8 | 1.4 | 2 | 50 | 0.01 | 4.16 | 26.56 | 7.18 | 59.84 | 2.25 |
| 9 | 3 | 2 | 50 | 0.03 | 13.97 | 74.21 | 1.51 | 7.95 | 2.34 |
| 10 | 3 | 2 | 50 | 0.03 | 15.45 | 76.78 | 0.87 | 3.93 | 2.93 |
| 11 | 3 | 2 | 50 | 0.03 | 16.18 | 80.96 | 0.14 | 0.29 | 2.41 |
| 12 | 2 | 3 | 40 | 0.02 | 6.64 | 39.34 | 5.93 | 47.38 | 0.70 |
| 13 | 3 | 2 | 50 | 0.02 | 16.17 | 81.26 | 0.37 | 1.08 | 1.09 |
| 14 | 4 | 1 | 60 | 0.04 | 15.68 | 78.34 | 1.12 | 3.62 | 1.21 |
| 15 | 4 | 1 | 40 | 0.02 | 12.62 | 71.37 | 2.40 | 11.88 | 1.71 |
| 16 | 2 | 1 | 40 | 0.02 | 12.37 | 69.71 | 2.60 | 13.37 | 1.93 |
| 17 | 4 | 3 | 40 | 0.02 | 16.71 | 82.56 | 0.01 | 0.03 | 0.67 |
| 18 | 4 | 3 | 60 | 0.03 | 16.77 | 82.37 | 0.01 | 0.01 | 0.81 |
| 19 | 2 | 3 | 60 | 0.01 | 6.24 | 37.46 | 5.95 | 47.95 | 2.40 |
| 20 | 2 | 1 | 60 | 0.04 | 13.55 | 73.44 | 1.83 | 7.59 | 3.56 |

As shown in Table 1, as a result of carrying out the reaction with an initial equivalent of NaBH₄ of 2.0 equiv, the same level of the product conversion was observed at 2.5 mol% and 5 mol% (entry 2 and 3). When comparing the results in which about 29% of the reactant ("SG10") remained under the two conditions of 2.5 mol% and 5.0 mol%, it was confirmed that the acid by-product (RRT14.7), which was removed in the subsequent purification process, was 0.6% and 2.6%, respectively, and was higher under the condition of 5.0 mol%. Based on these results, it was determined that using 2.5 mol% of NaOMe was preferable in terms of product purity. In the above experiment, the reaction was not completed even after 16 hours, so the reaction was carried out using 3.0 equiv of NaBH₄ under the condition of 2.5 mol% of NaOMe, and it was confirmed that the reaction was completed.

Table 2 shows the evaluation result of the reaction yield, depending on the equivalent of NaBH4 and the amount (fold) of MeOH used when the amount of NaOMe used was fixed to 2.5 mol%. Based on the result in Table 2, it was confirmed that when 3.0 equiv of NaBH4 and 1.6-fold of MeOH were used, the reaction could be completed without additional addition of NaBH4 during the reduction reaction and be completed at a temperature of less than 60°C, in particular, the temperature of 50°C.

As described above, in the reduction reaction of (3-chloro-1-isopropyl-1H-indazol-5-yl)-methanol, it was confirmed that using an alkoxide additive could solve the problem of lowering the process stability due to the additional addition process of a reducing agent and control the generation of hydrogen gas during the reaction.

### Experimental Example 2. Evaluation of purity improvement of the product by acidification purification after reduction reaction of (3-chloro-1-isopropyl-1H-indazol-5-yl)-methanol

When the reactant is over-reduced during the reduction reaction of (3-chloro-1-isopropyl-1H-indazol-5-yl)-methanol, there is a problem in that an impurity (RRT 0.88 IMP) in which the olefin group within the indole structure of the product has been further reduced is formed.

It was confirmed that these impurities could be effectively decreased through the purification process under an acidic condition after completion of the reduction reaction. The results are shown in Tables 3 and 4 below.

**[Table 3]**

| Run | W/U conditio n | Temp (°C) | Time (Hour ) | Layer | SG15 HPLC (% PAR) - 288 nm | | | | |
|---|---|---|---|---|---|---|---|---|---|
| | | | | | RRT 0.88 | RRT 0.91 | SG15 | 1.44 | New IMP. * (benz yl-Cl) |
| Ini. | - | - | - | Org. | 0.08 | 8.52 | 87.11 | 0.48 | 0.02 |
| 1 | 6N HCl | 20 | 0.5 | Org. | 0.02 | 0.10 | 89.50 | | 7.00 |

**[Table 4]**

| Run | W/U solvent | Temp (°C) | Time (Hour ) | Layer | SG15 HPLC (% PAR) - 288 nm | | | | |
|---|---|---|---|---|---|---|---|---|---|
| | | | | | RRT 0.88 | RRT 0.91 | SG15 | 1.44 | New IMP. * (benz yl-Cl) |
| Ini. | - | - | - | Org. | 0.08 | 14.58 | 78.28 | 0.31 | N/D |
| 1 | Toluene | 0 | 0.5 | Org. | 0.004 | 0.25 | 94.73 | 0.39 | 0.19 |
| 2 | EtOAc | 0 | 0.5 | Org. | 0.01 | 0.57 | 92.84 | 0.47 | 0.10 |
| 3 | Me-THF | 20 | 0.5 | Org. | 0.05 | 11.19 | 81.53 | 0.34 | 0.01 |
| 4 | MTBE | 20 | 0.5 | Org. | 0.01 | 0.96 | 91.9 | 0.66 | 0.08 |

As shown in Table 3 above, as a result of washing with hydrochloric acid, the over-reduction impurity (RRT 0.88 impurity) decreased from initially 0.08% to 0.02%, and the N2 isomer (RRT 0.91 impurity) reduced to 0.1%. However, a new impurity (New IMP. benzyl-Cl) expected to be benzyl-chloride was found. Table 4 shows the purification result depending on the extraction solvent when the purification process is performed using 6 N hydrochloric acid. As shown in Table 4 above, it was confirmed that impurities of RRT 0.88 and RRT 0.91 could be most effectively removed when toluene was used as the extraction solvent during the purification process, and the generation of the new impurity expected to be benzyl chloride could also be inhibited.

### Experimental Example 3. Evaluation of solvents in the substitution reaction in the synthesis of 3-chloro-1-isopropyl-1H-indazole-5-carboxylic acid methyl ester

In the conventional substitution reaction of chloride and isopropyl for 1H-indazole-5-carboxylic acid methyl ester in dimethylformamide (DMF) solvent, a problem was found that an impurity (RRT 1.44) is formed due to the chloride-adduct (Cl-adduct), and the impurity is not removed in the subsequent process and remains in the final product. In addition, when the internal temperature increases rapidly due to exotherm during the substitution reaction of 1H-indazole-5-carboxylic acid methyl ester with chloride and isopropyl, a tendency to increase the amount of the impurity was observed.

Accordingly, the same reaction was performed by changing the reaction solvent to dimethylacetamide (DMA), and the result was compared and evaluated with the yield when the reaction was performed in DMF. The result is shown in Table 5 below.

In Table 5 below, "SG01" represents "1H-indazole-5-carboxylic acid methyl ester", and "SG05" represents "methyl 3-chloro-1H-indazole-5-carboxylate".

**[Table 5]**

| solvent | Temp (°C) | Time (hour) | SG05 HPLC (% PAR) | |
|---|---|---|---|---|
| | | | SG01 residue | RRT 1.44 |
| DMF | 65 | 2 | 0.35 | 1.07 |
| DMA | 65 | 2 | 0.26 | 0.4 |

As shown in Table 5 above, it was confirmed that the purity of the reaction product could be increased by changing the reaction solvent to DMA, accordingly increasing the degree of completion of the reaction, and remarkably reducing the amount of impurity generated. It is inferred that this is because DMA has a relatively low decomposition rate, whereas there is a problem in that DMF is decomposed at high temperatures to produce dimethylamine, and as the produced dimethylamine reacts with NCS, heat generates. To verify the above result, a stabilization study was performed additionally. Table 6 shows the results of measuring the temperature changes for the maximum temperature of synthesis reaction (MTSR), which is the maximum temperature attained during the synthesis reaction, and for the resulting adiabatic temperature rise (ΔTad) when each DMF or DMA is added to 1H-indazole-5-carboxylic acid methyl ester and NCS and the temperature is raised to 65°C.

**[Table 6]**

| Solvent | Reaction Temperatu re (°C) | Maximum Temperatur e of Synthesis Reaction (MTSR, °C) | Adiabatic Temperatu re Rise (ΔTad, K) | Maximum Technical Temperatu re (MTT, °C) | Heat of Reaction (qr_hf, kJ) |
|---|---|---|---|---|---|
| DMF | 65 | 90.8 | 25.8 | 153 | 20.2 |
| DMA | 65 | 67.6 | 2.6 | 165 | 9.8 |

As shown in Table 6, when DMF was added, the maximum temperature of synthesis reaction was measured to be 91.5°C, and the adiabatic temperature rise was measured to be 26.5K. When DMA was added, the maximum temperature of synthesis reaction was measured to be 67.9°C, and the adiabatic temperature rise was measured to be 2.9K. In addition, when DMF was added, the heat value was measured to be 20.2 kJ, and when DMA was added, the heat value was measured to be 9.8 kJ. It means that when DMF is used as the reaction solvent in the above process, the temperature rise due to the synthesis reaction is larger, and the thermal risk is higher than when DMA is used. That is, it means that when DMF is used, a lot of reaction heat is generated from the synthesis reaction.

Accordingly, it is confirmed that to reduce RRT1.44 and enhance the purity of the product, it is preferable to use DMA rather than DMF as the reaction solvent, whereby the production of RRT1.44 could be controlled to be at 0.5% or less and could be controlled at a level of 0.10% or less impurities in 5-bromomethyl-3-chloro-1-isopropyl-1H-indazole, which is the final API.

## Claims

1. A method for preparing an intermediate compound of Formula 4 below, comprising: 1) a step for performing a reduction reaction of a compound of Formula 2 in the presence of an alcohol-based solvent and an alkoxide to obtain a compound of Formula 3; and
2) a step for substituting the alcohol group of the compound of Formula 3 with a leaving group: 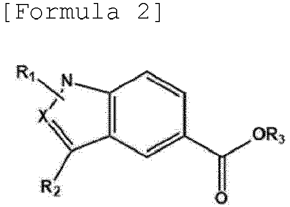 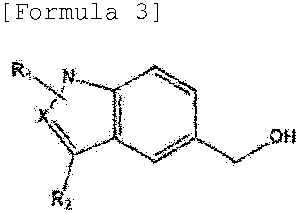 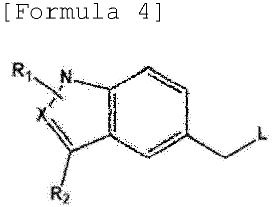 wherein
R1 is hydrogen or a substituted or unsubstituted alkyl,
R2 is hydrogen, a substituted or unsubstituted alkyl, a halogen, CN, CF₃, or COCF₃,
R3 is a substituted or unsubstituted alkyl,
X is C or N, and
L is a leaving group.

2. The preparation method of claim 1, wherein R1 is a C₁-C₄ substituted or unsubstituted alkyl,
R2 is a halogen,
R3 is a C₁-C₄ substituted or unsubstituted alkyl, and
L is a leaving group selected from among chlorine (Cl), bromine (Br), iodine (I), methanesulfonate (Oms), p-toluenesulfonate (OTs), and trifluoromethanesulfonate (OTf).

3. The preparation method of claim 1, wherein in step 1), the alkoxide includes one having the same alkyl group as the alkyl group of the alcohol-based solvent.

4. The preparation method of claim 3, wherein in step 1), the alcohol-based solvent includes methanol, and the alkoxide includes methoxide.

5. The preparation method of claim 1, wherein in step 1), the alkoxide includes the same metal ion as the metal ion within a reducing agent used in the reduction reaction.

6. The preparation method of claim 1, wherein in step 1), the reducing agent is selected from among sodium borohydride (NaBH₄), lithium borohydride (LiBH₄), borane (BH₃), and diisobutylaluminum hydride (DIBAH).

7. The preparation method of claim 1, wherein step 1) further comprises purifying under an acidic condition after completion of the reduction reaction.

8. The preparation method of claim 7, wherein the purifying is performed at a temperature of 0°C to 20°C.

9. The preparation method of claim 7, wherein the purifying is performed in the presence of an organic solvent containing toluene.

10. The preparation method of claim 1, wherein the compound of Formula 2 is prepared by introducing R1 and R2 substituents to a compound of Formula 5 below: 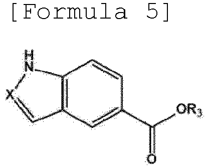 wherein
R1, R2, R3, and X are as defined in claim 1.

11. The preparation method of claim 10, wherein the reactions for introducing the substituents are performed in the presence of dimethylacetamide (DMA) solvent.
